# EUROPEAN PATENT SPECIFICATION

(11) **EP 3 262 156 B1**
(45) Date of publication and mention of the grant of the patent: **23.06.2021**
(21) Application number: 16719951.2
(22) Date of filing: 26.02.2016
(51) Int. Cl.: C12N 5/071, A01N 1/02, C12Q 1/02

(54) **METHOD FOR POOLING HEPATOCYTES**
VERFAHREN ZUM POOLING VON HEPATOZYTEN
MÉTHODE DE MISE EN COMMUN D'HÉPATOCYTES

(30) Priority: 27.02.2015 US 201562121619 P
(43) Date of publication of application: 03.01.2018
(73) Proprietor: Lonza Walkersville, Inc., Walkersville, MD 21793 (US)
(72) Inventor: KAISER, Robert, Fuquay Varina,NC 27526 (US); SHERMAN, Matthew, Raleigh, NC 27614 (US)
(74) Representative: Gassner, Birgitta
(86) International application number: PCT/US2016/019742
(87) International publication number: WO 2017/119917

(56) References cited:
- WO-A2-2014/045202
- US-B2- 7 604 929
- LI A P: "Application of cryopreserved human hepatocytes in drug development: Metabolism, drug-drug interactions, and drug toxicity", CURRENT TOPICS IN PHARMACOLOGY, vol. 17, no. 2, 2013, pages 47-66, XP008182211, TRIVANDRUM, INDIA ISSN: 0972-4559
- STÉPHENNE X ET AL: "Hepatocyte cryopreservation:Is it time to change the strategy?", WORLD JOURNAL OF GASTROENTEROLOGY, vol. 16, no. 1, 7 January 2010 (2010-01-07), pages 1-14, XP055214777, ISSN: 1007-9327, DOI: 10.3748/wjg.v16.i1.1
- SUMIDA K ET AL.: "Effects of DMSO on gene expression in human and rat hepatocytes", HUMAN AND EXPERIMENTAL TOXICOLOGY, vol. 30, no. 10, 2011, pages 1701-1709,

## Description

### FIELD OF THE INVENTION

The present invention relates generally to pooling and cryopreserving hepatocytes from multiple donors.

### BACKGROUND

Hepatocytes constitute approximately 80% of the cells in the liver and are critical for both the activation and eventual detoxification of many pharmacological compounds, toxins or xenobiotics. Increased demand for and availability of new drugs, as well as stricter regulatory and safety testing prior to market approval, have made isolated primary hepatocytes an invaluable resource for studying drug metabolism, efficacy and toxicity in a laboratory setting.

In recent years, significant advancements have been made in the isolation and cryopreservation of primary donor hepatocytes, which can be rapidly thawed and immediately used for experimentation. However, studying hepatic metabolism in hepatocytes isolated from one human liver (individual donor) does not accurately reflect liver function in the overall population since variations in gender, age, ethnicity, health status, genetic background, and other factors skew test results. A more accurate measure of hepatic metabolism is to a use a mixture or "pool" of individual donor cells to create a heterogeneous population of hepatocytes.

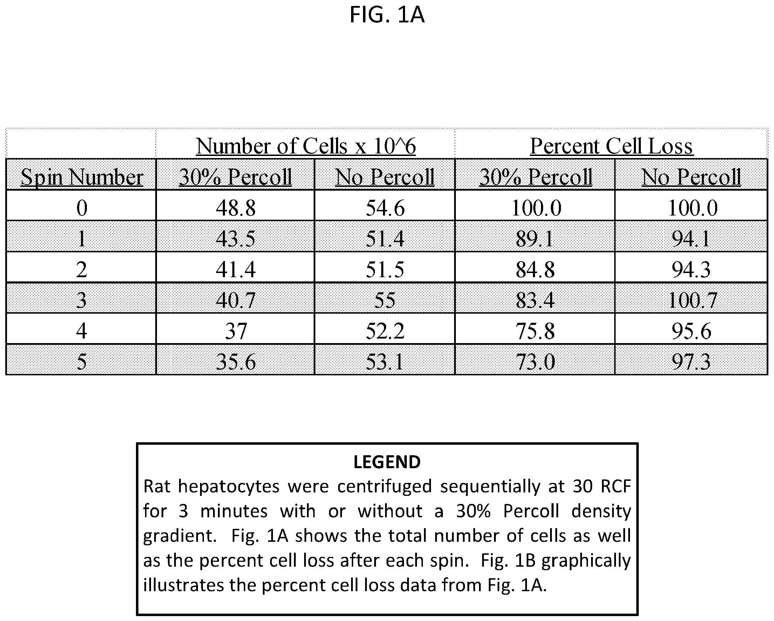

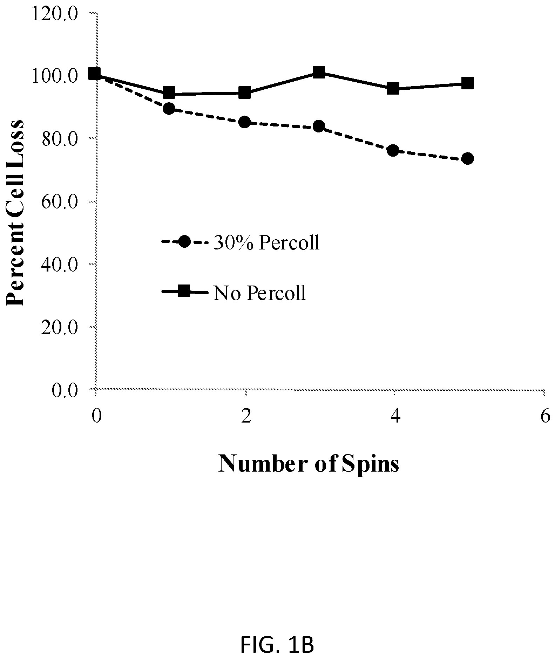

A number of methods have been proposed for pooling hepatocytes. These protocols often employ lengthy procedures in which cells are exposed to both physical and chemical stress that reduce the total number of viable cells. For example, the method in U.S. Pat. 7,604,929 utilizes a density gradient centrifugation step before the second or final cryopreservation step. This subjects the cells to chemical and mechanical stress that either results in cell loss (Figs. 1A-B) or weakens the cells such that they die during cryopreservation. Another method disclosed in WO 2014/045202 A2 maintains the hepatocytes in a cryopreservative solution throughout the pooling process. Cryopreservative solutions contain toxic reagents, such as dimethyl sulfoxide (DMSO), which are known to cause cell death.

**The** proposed system and method seeks to employ techniques to reduce cell loss and therefore increase the total number of viable cells throughout the process.

### SUMMARY

The application seeks to increase the number of resulting viable cells from a hepatocyte pooling process. One method for cryopreserving hepatocytes from multiple sources comprises the steps of:
A) thawing hepatocytes from a plurality of sources preserved in a cryopreservation solution comprising DMSO;
B) pooling 300 to 1000 vials of hepatocytes from the plurality of sources into a preservation solution, thereby diluting the concentration of DMSO;
C) centrifuging the pooled hepatocytes to cause pelleting of both viable and non-viable hepatocytes;
D) removing the preservation solution;
E) combining the viable and non-viable pelleted hepatocytes with a cryopreservative;
F) distributing the pooled hepatocytes into vials; and
G) cryopreserving the hepatocytes in the vials.

The above method can utilize different kinds of hepatocytes including those selected from the group comprising: human hepatocytes, porcine hepatocytes, simian hepatocytes, canine hepatocytes, feline hepatocytes, bovine hepatocytes, equine hepatocytes, ovine hepatocytes and rodent hepatocytes.

The individual sources can be pooled based on gender, race, age, metabolic state or health state. In some instances, the pools can be randomized based on the sample set that exists at the time.

The kinds of preservation solution that can be used include: University of Wisconsin solution, HypoThermosol-Base, or HypoThermosol-FRS as well as other similar preservation solutions that reduce the toxicity of the cryopreservative and/or provide essential nutrients for the hepatocytes. For example, the preservation solution could include fetal bovine serum.

The centrifugation step is devoid of a density gradient to reduce physical and chemical stress on the cells. This centrifugation step pellets both viable and non-viable hepatocytes.

**The** pellets can be combined with a cryopreservative prior to being frozen. When combining the cryopreservative and the pelleted cells, pipetting up and down, vortexing, rocking the vial back and forth, tapping the vial or similar processes can be used to resuspend the cells in the cryopreservative solution.

When distributing the pelleted hepatocytes into vials via aliquoting or other methods, the pooled cells can be distributed at a density that ranges from 8-15 million cells/ml. In one embodiment, 13.33 million cells/ml is used.

After the final thaw of the pooled hepatocytes, a user can perform density gradient fractionation to separate viable and non-viable cells immediately prior to performing experiments. In some instances, the density gradient centrifuging step is performed between 50-200 RCF. The density gradient fractionation can comprise density gradient centrifugation through polyvinylpyrrolidone-coated colloidal silica particles (Percoll).

The hepatocytes used in these processes can either be plated or used in suspension.

### BRIEF DESCRIPTION OF THE DRAWINGS

Figs. 1A-B illustrate the yield of viable rat hepatocytes after sequential centrifugation with or without a Percoll gradient.
Fig. 2 is a schematic of a method for pooling and cryopreserving hepatocytes from previously cryopreserved hepatocytes from individual donors.

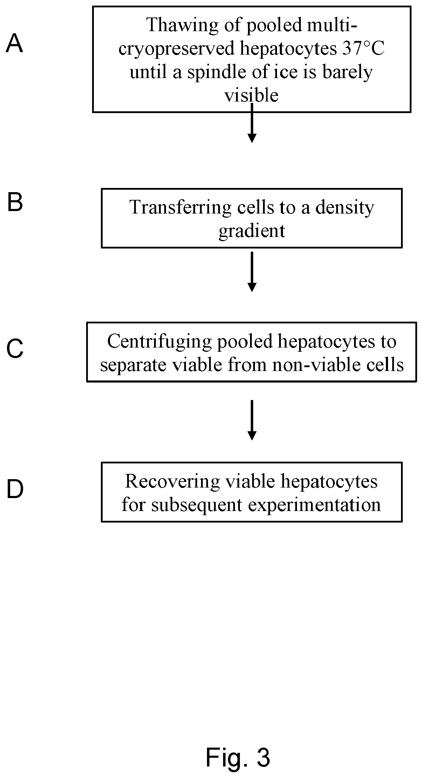

Fig. 3 is a schematic of a method to separate viable from non-viable cells from the last cryopreservation step of Fig. 2.

### DETAILED DESCRIPTION

**A** description of an improved system and method for preparing a cryopreserved pool of cells (e.g. hepatocytes) from previously cryopreserved hepatocytes obtained from individual donors is described herein. This system and method reduces physical and chemical stress to the hepatocytes during the pooling process while increasing the recovery of viable pooled cells during a post-thaw centrifugation step performed by the end-user. In general, this system and method involves thawing vials of individual donor hepatocytes and pooling them into a preservation solution. The pooled cells are then briefly centrifuged, to pellet both viable and non-viable cells, and then cryopreserved at a high density in multiple vials. The end-user can then perform density gradient centrifugation to separate viable from non-viable cells immediately prior to experimental use. Some of the advantages of the proposed method are decreasing the exposure to mechanical, chemical and other environmental factors that reduce the number of viable cells prior to experimenting on the pooled hepatocytes.

In one embodiment, previously isolated and cryopreserved hepatocytes from individual donors are stored in liquid nitrogen vapor phase at a minimum of -150°C. Individual donor vials to be pooled can be selected at random or based on specific metabolic activity (e.g. ECOD, cytochrome P450, general phase I or phase II), age, race, sex, ethnicity, or other phenotypic determinants. The number of vials thawed depends on the number of individuals included in the mixed population and the size of the pool to be generated. For example, the number of individual vials used for each pool of hepatocytes can be from 2 - 50 individuals. Each pool can range from 300 to 1000 vials. For example, a 10 donor pool with 300 vials would require approximately 30 vials from each donor.

**The** individual donor hepatocytes are thawed by submerging the vial in a water bath maintained at 37°C for approximately 2 minutes or until a spindle of ice is barely visible and then the contents are rapidly decanted into a vessel containing a preservation solution at 4°C. The preservation solution is used to dilute the DMSO (and/or any other reagents toxic to the hepatocytes) found in the cryopreservative. In some instances, the preservation solution also provides essential nutrients to the hepatocytes to promote viability during the pooling process. The volume of the preservation solution is dependent on the size of the pool to be generated but can be composed of, for example, a 1:1 ratio of preservation solution to 1 mL of cryopreserved cells. The preservation solution can be composed of University of Wisconsin solution (10 mM potassium lactobionate, 25 mM KH2PO4, 5 mM MgSO4, 30 mM Raffinose, 5 mM Adenosine, 3 mM Glutathione, 1 mM Allopurinol, and 50 g/L Hyroxyethyl starch), HypoThermosol-Base (HTS-Base), HypoThermosol-FRS (HTS-FRS), or other such medium with or without the addition of fetal bovine serum (FBS). The hepatocytes are maintained in the preservation solution at 4°C while multiple vials of hepatocytes are thawed and mixed in the same vessel to create a "pooled" population of multiple donor cells. Thawed hepatocytes can remain in preservation solution from 2 - 10 hours depending on the number of vials being thawed.

After multiple vials of hepatocytes have been thawed and pooled, the cells are centrifuged in a range of 50 - 200 relative centrifugal force (RCF) for 8 - 10 min to pellet both viable and non-viable cells. The preservation solution is removed from the pelleted cells for example by aspiration. Using a centrifugation step without a density gradient at this point in the process, reduces the physical and chemical stress on the hepatocytes before the second cryopreservation. Figs. 1A-B illustrate the affect that density gradient (e.g. Percoll) centrifugation can have on hepatocytes compared to centrifugation without a density gradient. With each subsequent spin, the total number of viable cells was reduced with the density gradient (Percoll) relative to hepatocytes centrifuged without Percoll. These results indicate that centrifugation with a density gradient solution reduces the total number of viable cells.

After the cells are pelleted and the preservation solution removed, they are immediately resuspended in a cryprotectant solution which can, for example, be composed of CryoStor® CS10 containing DMSO at 10%. The pooled non-viable and viable hepatocytes are then distributed at 10-15 million cells / mL in 1 - 1.5 mL aliquots per vial. It should be noted that cell counting can be performed without or with for example Trypan blue, Acridine orange, or propidium iodide along any of the steps in the process.

The combining or resuspending process can be comprised of adding a cryopreservative to the pelleted hepatocytes. The combined pellets and cryopreservative can then be pipetted up and down, vortexed, rocked in a vial back and forth, tapped in a vial or some other similar process to break up the pellets and disperse the hepatocytes throughout the cryopreservative.

Vials containing pooled hepatocytes are frozen using a controlled rate freezer and maintained in liquid nitrogen at a minimum of -150°C for at least 3 days and no longer than 10 years prior to shipping. The vials can be shipped on dry-ice or vapor phase liquid nitrogen (e.g. dewar) to the end-user and stored in liquid nitrogen at a minimum of -150°C. Immediately prior to use, the end-user can thaw the pooled hepatocytes by submerging the vial in a water bath maintained at 37°C for approximately 2 minutes or until a spindle of ice is barely visible.

The pooled non-viable and viable hepatocytes cells can be applied to a 20 - 30% colloidal silica coated with polyvinylpyrrolidone (Percoll) gradient, and centrifuged through at 50 - 200 RCF for 8-10 min to separate viable from non-viable cells. The maximum number of viable cells are therefore isolated immediately prior to use without further exposure to cryopreservative or another freeze-thaw cycle. This process allows for 5 million and up to 8+ million viable cells that can be recovered and immediately used for experimentation. Experimentation may include but not be limited to assays for viability; metabolic activity; transporter activity; and xenobiotic uptake, metabolism, efficacy, and toxicity.

### EXAMPLE 1

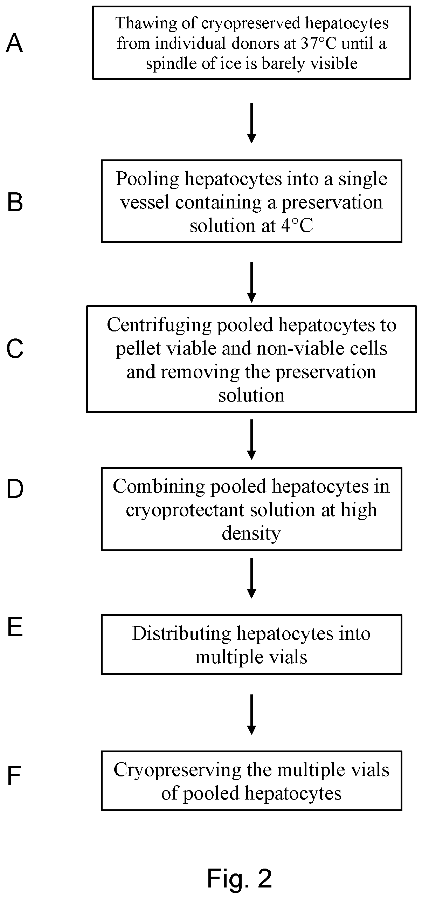

Procedure for the preparation of pooled hepatocytes from individual cryopreserved donor hepatocytes are shown in Figure 2 and set forth in the following operation:
1-A. Vials of cryopreserved hepatocytes from 10 individual donors are thawed for approximately 2 minutes in a 37°C water bath until a spindle of ice is barely visible. It should be noted, some pools can be composed of between 500 and 900 vials, or in this example 50 to 90 vials per donor.
1-B. The thawed hepatocyte suspension (1 mL) is pipetted into a 1 L beaker containing 500 mL of HypoThermosol-FRS preservation solution and maintained at 4°C to generate a hepatocyte pool.
1-C. The pooled hepatocytes are centrifuged at 100G for 10 minutes out of the preservative solution (FRS) to pellet both viable and non-viable cells.
1-D. The preservation solution is removed by aspiration and the cells are gently resuspended (e.g. rocked back and forth) in the cryoprotectant CryoStor® CS10 medium. Cells are counted using Trypan blue exclusion to determine cells density and additional cryoprotectant solution is added, if needed, to achieve approximately 13.3 x10^6 cells per mL.
1-E. 1.5 milliliters or approximately 20 million cells are aliquoted into individual vials.
1-F. The vials of pooled hepatocytes are cryopreserved in a controlled rate freezer and stored in liquid nitrogen vapor phase at a minimum of -150°C.

Procedure for the separation of viable cells from pooled hepatocytes as performed by the end-user are show in Figure 2 and set forth in the following operation:
2-A. A vial of pooled hepatocytes is thawed for approximately 2 minutes in a 37°C water bath until a spindle of ice is barely visible.
2-B. The hepatocyte suspension is carefully applied to a 20-30% Percoll density gradient
2-C. The samples are centrifuged at 200 RCF for 10 min to separate viable and non-viable cells
2-D. A minimum of 5 million viable cells are recovered and used immediately for experimentation

It should be noted that yield of viable cells can vary with dilution ratios, and exposure of time during the pooling process. For example, in larger batches it is sometimes cost prohibitive or difficult (volume of pooling container) to have a 1:1 ratio of preservation solution to thawed cryopreservative. In addition, more time is required to thaw a large number of vials. Thus the cells are exposed to increased concentrations of cryopreservative toxins for a longer period of time. It is sometimes easier to do smaller batches, in which the cryopreservative is more dilute and pooling time is reduced, which generally results in higher yields of viable hepatocytes. For example, step 2-D might yield 5 million viable cells for a larger batch, but up to and greater than 8 million viable cells in a smaller batch. In smaller batches the ratio may be 4:1 preservation solution to thawed cryopreservative.

The processes herein can be applied to hepatocytes used in suspension or plated.

Though the examples describe hepatocytes the method and processes could be applied to other cell types.

These embodiments and features illustrated and described herein are exemplary but not intended to be limiting nor are the claims listed at the end of this application. Multiple combinations and equivalent component parts, ranges and steps are considered within the scope of this application.

## Claims

1. A method for cryopreserving hepatocytes from multiple sources comprising the steps of:
A) thawing hepatocytes from a plurality of sources preserved in a cryopreservation solution comprising DMSO;
B) pooling 300 to 1000 vials of hepatocytes from the plurality of sources into a preservation solution thereby diluting the concentration of DMSO;
C) centrifuging the pooled hepatocytes to cause pelleting of both viable and non-viable hepatocytes;
D) removing the preservation solution;
E) combining the viable and non-viable pelleted hepatocytes with a cryopreservative;
F) distributing the pooled hepatocytes into vials
G) cryopreserving the hepatocytes in the vials

2. The method of claim 1, wherein said hepatocytes are selected from the group comprising: human hepatocytes, porcine hepatocytes, simian hepatocytes, canine hepatocytes, feline hepatocytes, bovine hepatocytes, equine hepatocytes, ovine hepatocytes and rodent hepatocytes

3. The method of claim 1, wherein said multiple sources are comprised of a random or pre-selected group based on gender, race, age, metabolic state or health state.

4. The method of claim 1 , wherein the preservation solution is comprised of University of Wisconsin solution, HyperThermosol-Base, or HyperThermosol-FRS.

5. The method of claim 4, wherein the preservation solution is further comprised of fetal bovine serum.

6. The method of claim 1, wherein distributing the pooled cells is done at a density greater than 10 million cells/ml.

7. The method of claim 1, wherein the centrifugation step is devoid of a density gradient.

8. The method of claim 1 , wherein the centrifugation step is performed to pellet both viable and non-viable cells.

9. The method of claim 1, further including thawing the pooled cryopreserved cells and applying a density gradient fractionation process.

10. The method of claim 9, wherein the density gradient centrifuging step is performed between 50-200 RCF.

11. The method of claim 9, wherein said density gradient fractionation comprises density centrifugation through polyvinylpyrrolidone-coated colloidal silica particles.

12. The method of claim 2, wherein the hepatocytes are either used in suspension or plated.

13. The method of claim 1 , wherein the combining of pelleted hepatocytes with cryopreservative further includes one or more of the following steps: pipetting up and down, vortexing, rocking vial back and forth, or tapping the vial.

14. The method of claim 1, wherein the thawed hepatocytes remain in the preservation solution during pooling in step B for 2 to 10 hours.

## Patentansprüche

1. Verfahren zur Kryokonservierung von Hepatozyten aus mehreren Quellen, umfassend die folgenden Schritte:
A) Auftauen der Hepatozyten aus einer Vielzahl von Quellen, die in einer Kryokonservierungslösung konserviert werden, die DMSO enthält;
B) Poolen von 300 bis 1000 Ampullen mit Hepatozyten aus der Vielzahl von Quellen in einer Konservierungslösung, wodurch die Konzentration von DMSO verdünnt wird;
C) Zentrifugieren der gepoolten Hepatozyten, um eine Pelletierung sowohl lebensfähiger als auch nicht lebensfähiger Hepatozyten zu bewirken;
D) Entfernen der Konservierungslösung;
E) Kombinieren der lebensfähigen und nicht lebensfähigen pelletierten Hepatozyten mit einem Kryokonservierungsmittel;
F) Aufteilen der gepoolten Hepatozyten auf Ampullen;
G) Kryokonservieren der Hepatozyten in den Ampullen.

2. Verfahren nach Anspruch 1, wobei die Hepatozyten aus der Gruppe ausgewählt werden, umfassend: menschliche Hepatozyten, Schweine-Hepatozyten, Affen-Hepatozyten, Hunde-Hepatozyten, Katzen-Hepatozyten, Rinder-Hepatozyten, Pferde-Hepatozyten, Schaf-Hepatozyten und Nager-Hepatozyten.

3. Verfahren nach Anspruch 1, wobei die mehreren Quellen aus einer auf Basis von Geschlecht, ethnischer Zugehörigkeit, Alter, Stoffwechselzustand oder Gesundheitszustand zufällig oder vorab ausgewählten Gruppe bestehen.

4. Verfahren nach Anspruch 1, wobei die Konservierungslösung aus einer University-of-Wisconsin-Lösung, HyperThermosol-Base oder HyperThermosol-FRS besteht.

5. Verfahren nach Anspruch 4, wobei die Konservierungslösung ferner aus fötalem Rinderserum besteht.

6. Verfahren nach Anspruch 1, wobei das Aufteilen der gepoolten Zellen mit einer Dichte von mehr als 10 Millionen Zellen/ml erfolgt.

7. Verfahren nach Anspruch 1, wobei beim Zentrifugierschritt kein Dichtegradient angewendet wird.

8. Verfahren nach Anspruch 1, wobei der Zentrifugierschritt durchgeführt wird, um sowohl lebensfähige als auch nicht lebensfähige Zellen zu pelletieren.

9. Verfahren nach Anspruch 1, ferner beinhaltend das Auftauen der gepoolten kryokonservierten Zellen und das Anwenden eines Dichtegradienten-Fraktionierungsvorgangs.

10. Verfahren nach Anspruch 9, wobei der Dichtegradienten-Zentrifugierschritt mit 50 bis 200 RCF durchgeführt wird.

11. Verfahren nach Anspruch 9, wobei die Dichtegradienten-Fraktionierung eine Dichtezentrifugierung durch mit Polyvinylpyrrolidon beschichteten kolloidale Kieselerdepartikel umfasst.

12. Verfahren nach Anspruch 2, wobei die Hepatozyten entweder in Suspension oder plattiert verwendet werden.

13. Verfahren nach Anspruch 1, wobei das Kombinieren von pelletierten Hepatozyten mit einem Kryokonservierungsmittel ferner einen oder mehrere der folgenden Schritte beinhaltet: Auf- und Abpipettieren, Verwirbeln, Hin- und Herschwenken der Ampulle oder Klopfen auf die Ampulle.

14. Verfahren nach Anspruch 1, wobei die aufgetauten Hepatozyten während des Poolens in Schritt B 2 bis 10 Stunden in der Konservierungslösung bleiben.

## Revendications

1. Procédé de cryoconservation d'hépatocytes de sources multiples comprenant les étapes consistant à :
A) décongeler les hépatocytes d'une pluralité de sources conservés dans une solution de cryoconservation comprenant du DMSO ;
B) réunir 300 à 1000 fioles d'hépatocytes de la pluralité de sources dans une solution de conservation diluant ainsi la concentration de DMSO ;
C) centrifuger les hépatocytes réunis pour entraîner le culottage à la fois des hépatocytes viables et non viables ;
D) enlever la solution de conservation ;
E) combiner les hépatocytes culottés viables et non viables avec un cryoconservateur ;
F) distribuer les hépatocytes réunis dans des fioles ;
G) cryoconserver les hépatocytes dans les fioles.

2. Procédé selon la revendication 1, dans lequel lesdits hépatocytes sont choisis dans le groupe comprenant : des hépatocytes humains, des hépatocytes porcins, des hépatocytes simiens, des hépatocytes canins, des hépatocytes félins, des hépatocytes bovins, des hépatocytes équins, des hépatocytes ovins et des hépatocytes de rongeur

3. Procédé selon la revendication 1, dans lequel lesdites multiples sources comprennent un groupe au hasard ou pré-sélectionné basé sur le genre, la race, l'âge, l'état métabolique ou l'état de santé.

4. Procédé selon la revendication 1, dans lequel la solution de conservation comprend en outre la solution de l'Université du Wisconsin, l'hyperthermosol-base ou l'hyperthermosol-FRS.

5. Procédé selon la revendication 4, dans lequel la solution de conservation comprend en oute du sérum bovin fœtal.

6. Procédé selon la revendication 1, dans lequel la distribution des cellules réunies est effectuée à une densité de plus de 10 millions de cellules/ml.

7. Procédé selon la revendication 1, dans lequel l'étape de centrifugation est sans gradient de densité.

8. Procédé selon la revendication 1, dans lequel l'étape de centrifugation est effectuée pour culotter à la fois les cellules viables et non viables.

9. Procédé selon la revendication 1, comprenant en outre la décongélation des cellules cryoconservées réunies et l'application d'un processus de fractionnement par gradient de densité.

10. Procédé selon la revendication 9, dans lequel l'étape de centrifugation par gradient de densité est effectuée entre 50 et 200 RCF.

11. Procédé selon la revendication 9, dans lequel ledit fractionnement par gradient de densité comprend une centrifugation de densité par des particules de silice colloïdale revêtues de polyvinylpyrrolidone.

12. Procédé selon la revendication 2, dans lequel les hépatocytes sont utilisés en suspension ou étalés.

13. Procédé selon la revendication 1, dans lequel la combinaison des hépatocytes culottés avec le cryoconservateur comprend en outre une ou plusieurs des étapes suivantes : pipetage de bas en haut, vortexage, basculement en avant et en arrière ou tapotage de la fiole.

14. Procédé selon la revendication 1, dans lequel les hépatocytes décongelés restent dans la solution de conservation durant la réunion dans l'étape B durant 2 à 10 heures.
